# EUROPEAN PATENT APPLICATION

(11) **EP 2 623 154 A1**
(43) Date of publication of application: **07.08.2013**
(21) Application number: 12154032.2
(22) Date of filing: 06.02.2012
(51) Int. Cl.: A61N 1/36

(54) **Neurostimulation System**

(71) Applicant: Sapiens Steering Brain Stimulation B.V., 5656 AE Eindhoven (NL)
(72) Inventor: Kleibeuker, Johan Gerard, 5268 HR Helvoirt (NL); Martens, Hubert Cécile François, 5611 ND Eindhoven (NL); Slobbe, Rob, 5263 EM Vught (NL)
(74) Representative: Rupprecht, Kay

(57) **Abstract**

The present invention relates to a neurostimulation system (10) comprising at least one electrode (120) configured such that a tissue, advantageously a brain tissue, can be stimulated, storage means for storing at least a first and a second set of electrophysiological data, whereby the first set of data is a set of intra-operatively acquired electrophysiological data and whereby the second set of data is a set of a post-operatively acquired electrophysiological data, comparison means configured such that the first set of data and the second set of data can be compared to receive a set of comparison data, and analyzing means configured such that the set of comparison data can be at least partially analyzed to determine the position of the at least one electrode (120) or to determine data, from which the position of the at least one electrode (120) can be determined.

## Description

The present invention relates to a neurostimulation system.

During the implantation of neurostimulation probes, the surgical team often performs a so-called electrophysiological mapping. Using acutely implanted test-probes, the functional properties of neuronal tissue is measured at different positions along the planned surgical trajectory towards the target area. This mapping is performed with the goal to accurately determine the spatial boundaries of the functional target region. Functional boundaries are identified from certain characteristic patterns in the recorded signals and further from analyzing the responses to test-stimulation. Based on the identification of functional boundaries the team decides upon the best location for positioning the final chronic probe.

New neurostimulation probes in development will have a large number (>10) of electrodes distributed around their surface. From these electrodes, the neurostimulation system may measure electrical neural activity and one may observe effects to test-stimulation from these electrodes. Such information may be helpful in the post-operative phase to identify electrodes that are close to the target region.

It is inevitable that in the post-operative phase the relative position of the chronic probe with respect to the target region changes. This can have various causes, e.g. that immediately following to the probe implantation the surgical handling (e.g. fixation) causes some mechanical forces on the probe resulting in displacements or that in the post-surgery recovery period, the tissue will deform (recover from brain-shift), resulting in a shift of tissue with respect to the probe or that probe-displacements may occur over time, especially after strong forces applied on the lead (wire) or head.

As a result of the above mechanisms, in general the implanted probe will not be at the presumed position as derived from the coordinates where the surgical team implanted the probe. This may make it more difficult to find the optimal stimulation settings (since the target position relative to the probe is uncertain).

It is therefore an object of the present invention to improve a neurostimulation system of the above-mentioned art, in particular in that the optimal stimulation settings can be found easier and with higher accuracy and further in that the position of an implanted chronic probe of a neurostimulation system can be determined with high accuracy.

This object is solved by the subject matter of claim 1 according to the present invention. Accordingly, a neurostimulation system comprises at least one electrode configured such that tissue, advantageously brain tissue, can be stimulated, storage means for storing at least a first and second set of electrophysiological data, whereby the first set of data is a set of intra-operatively acquired electrophysiological data and whereby the second set of data is a set of a post-operatively acquired electrophysiological data, comparison means configured such that the first set of data and the second set of data can be compared to receive a set of comparison data, and analyzing means configured such that the set of comparison data can be at least partially analyzed to determine the position of the at least one electrode or to determine data, from which the position of the at least one electrode can be determined.

Thereby, the advantage is achieved that the intra-operatively acquired information can be put in the right context of e.g. an implanted electrode within the brain and to accurately determine the position of the implanted electrode with respect to the target area. Consequently, a neurostimulation system can be improved, in particular in that the optimal stimulation settings can be found easier and with higher accuracy. In particular, functional boundaries determined during the implantation of a probe, in particular determined during surgery, can be placed in the context of the chronic situation of the implanted electrode, in particular at a later stage, and thus the target position with respect to the chronic electrode can be determined. Over the time, the electrode may get displaced and can have a different or unknown orientation with respect to the target area. By comparison of the first set of data and the second set of data the new electrode position can be derived.

In particular, the comparison of the first set of data and the second set of data to derive a new electrode position can be done as follows: The intra-operative measurement and the post-operative measurement is made with the same (chronic) probe carrying the at least one electrode. In this case the chronic probe is measured during surgical implantation with the goal to create an electrophysiological map (EP-map) of the target area. In the post-operative phase, from the same probe signals are recorded, e.g. by using an implantable pulse generator unit (IPG), and a post-op EP-map is constructed. By comparing the intra-op to the post-op EP-map, corrections can be made on target localization to the probe, e.g. to compensate a brain shift in the post-operative phase.

It is possible, that the data, from which the position of the at least one electrode can be determined are raw data including coordinates, angles and the electrophysiological data. It is also possible, that the data, from which the position of the at least one electrode can be determined also include recordings of neural activity, e.g. local field potential (LFP) and/or micro electrode recordings (MER) and that based on these data a so-called map of intra-operative electrophysiological data and a map of post-operatively electrophysiological data can be prepared and compared to each other.

The comparison means can be realized at least partially by a software program product, which is e.g. installed on a workstation being a part of the neurostimulation system or being connectable to the neurostimulation system.

Furthermore, the neurostimulation system may comprise at least one probe, whereby the probe is equipped with the at least one electrode.

Additionally, in a preferred embodiment it is possible that the probe is equipped with a plurality of electrodes, whereby the plurality of electrodes is arranged and predetermined to geometrical manner and whereby the electrodes are configured such that the electrodes are capable of providing electrical stimulation to the tissue and each of the electrodes are capable of detecting electrical signals. Thereby, the advantage is achieved that a tailor-made stimulation can be conducted by the probe which is equipped with a plurality of electrodes and that thereby the brain tissue to be stimulated can be stimulated with high accuracy. Additionally, the advantage is achieved that also electrical signals can be determined and that this determination process can be done with high accuracy.

Furthermore, the probe may be a chronic probe configured such that the probe is a long-term implantable probe.

It is additionally possible that the neurostimulation system may comprise test-probes which are intra-operatively acutely implantable. By this, the intra-operatively acquired data may be recorded.

The intra-operatively acutely implantable probes will only be used during surgery and the implantation process of e.g. the chronic probe or chronic probes and will be removed after the requisition of the intra-operatively electrophysiological data.

Preferably, the intra-operatively acquired data may comprise recordings of electrical brain activity from the at least one electrode, preferably the probes electrode, especially from a plurality of electrodes and/or stimulation-effects elicited by stimulation of the at least one electrode, preferably from a plurality of electrodes, especially from a group of pre-selected electrodes.

In a preferred embodiment, the plurality of electrodes is arranged on a chronic probe, whereby the electrodes are arranged in a predetermined geometrical manner forming an array on the probe. Additionally, the intra-operatively acquired data may be recorded either from acutely implanted test-probes or from the chronic probe or chronic probes during implantation or both.

Further preferably, the post-operatively acquired data comprise recordings of electrical brain activity from the at least one electrode, preferably the probes electrode, especially from a plurality of electrodes and/or stimulation-effects elicited by stimulation of the at least one electrode, preferably from a plurality of electrodes, especially from a group of pre-selected electrodes. Especially, the plurality of electrodes may be arranged on the chronic probe.

Additionally, the neurostimulation system may comprise controlling means configured such that the neurostimulation system can be controlled to acquire the at least first and second set of electrophysiological data.

In a further preferred embodiment it is possible that the neurostimulation system comprises intra-operative electrophysiology system means configured such that intra-operatively electrophysiological tissue characteristics at various positions in the brain can be captured.

Preferably, it is possible that the intra-operative electrophysiology system means comprise connection means configured such that intra-operative electrophysiology system means can be connected with the at least one electrode. Thereby, the intra-operative electrophysiology system means may receive data collected by the at least one electrode.

In a further preferred embodiment it is possible that the neurostimulation system comprises data processing means configured such that data captured by intra-operative electrophysiology system means can be analyzed and that at least partially the first set of data can be provided by the data processing means.

Further preferably, it is possible that the first set of data being a set of intra-operatively acquired electrophysiological data is a first 3D spatial map of local-field potential beta-activity and that the second set of data being a set of a post-operatively acquired electrophysiological data is a second 3D spatial map of local-field potential beta-activity.

Additionally, it is also possible that the neurostimulation system is configured such that the first 3D spatial map and the second 3D spatial map are compared by the comparison means and that the analyzing means are configured such that the analyzing means use a spatial translation to determine the position of the at least one electrode or to determine data, from which the position of the at least one electrode can be determined.

In a further preferred embodiment it is possible that the analyzing means are configured such that by means of the spatial translation functional boundaries determined intra-operatively are placed in the context of the chronic situation of the at least one electrode. By this, the advantage can be achieved that the new position of an implanted chronic probe can be determined, since it is possible that the implanted chronic probe may get displaced over the time.

Furthermore, it is possible that the analyzing means are configured such that the analyzing means use a spatial translation to process additional patient information data, especially an intra-operative image, preferably an image acquired by magnetic resonance imaging. Thereby, further data can be used to advantageously enhance the accuracy of the determination process of the position of the chronic probe.

Furthermore, in connection with the present invention as described above a method for determining a position of at least one electrode of a neurostimulation system is explicitly disclosed, whereby the at least one electrode is configured such that tissue, advantageously brain tissue can be stimulated, whereby at least a first and a second set of electrophysiological data is stored, whereby the first set of data is the set of intra-operatively acquired electrophysiological data and whereby the second set of data is the set of post-operatively acquired electrophysiological data, whereby the first and second set of data is compared to receive a set of comparison data and whereby the set of comparison data is analyzed at least partially to determine the position of the at least one electrode or determine data, from which the position of the at least one electrode can be determined.

As already disclosed above in connection with the neurostimulation system according to the invention, it is possible, that the data, from which the position of the at least one electrode can be determined are raw data including coordinates, angles and the electrophysiological data. It is also possible, that the data, from which the position of the at least one electrode can be determined also include recordings of neural activity, e.g. local field potential (LFP) and/or micro electrode recordings (MER) and that based on these data a so-called map of intra-operative electrophysiological data and a map of post-operatively electrophysiological data can be prepared and compared to each other.

In a first step of the method it is possible that at least one measurement probe is moved towards a target area and that different position measurements are taken. The target area can be an area in the brain, which is to be stimulated by a chronic probe of a neurostimulation system. This first step can be step, which is conducted intra-operatively.

In a second step of the method it is possible that from the different position measurements a spatial map of certain signal characteristics is created.

In a third step of the method it is possible that a chronic probe, e.g. a probe as defined above having a plurality of electrodes arranged on the chronic probe, whereby the electrodes are arranged in a predetermined geometrical manner forming an array on probe, is implanted at a certain position in the target area of the brain. The target area is the area to be stimulated by the chronic probe to conduct the neurostimulation therapy.

Since it is possible that over the time the chronic probe may get displaced and may have a different or unknown orientation with respect to the target area, in a fourth step a number of measurements is performed and a new map of signal characteristics is created.

In a fifth step it is possible that a transformation matrix is computed by comparing both maps. This transformation matrix may then be used to transform other data so that the new probe position can be derived.

The method can be conducted by using the neurostimulation system according to the present invention, especially a neurostimulation system according to any of the claims 1 to 15.

Further details and advantages of the present invention shall be described hereinafter with respect to the drawings:
- Figure 1:: the step of acquisition of the first set of data conducted by the neurostimulation system according to the present invention;
- Figure 2:: the step of creation of a spatial map conducted by the neurostimulation system according to the present invention;
- Figure 3:: the step of implantation of the chronic probe of the neurostimulation system according to the present invention;
- Figure 4:: a possible displacement of the chronic probe of the neurostimulation system according to the present invention;
- Figure 5:: the step of creation of a further spatial map conducted by the neurostimulation system according to the present invention; and
- Figure 6:: the step of computing a transformation map by comparing both spatial maps conducted by the neurostimulation system according to the present invention.

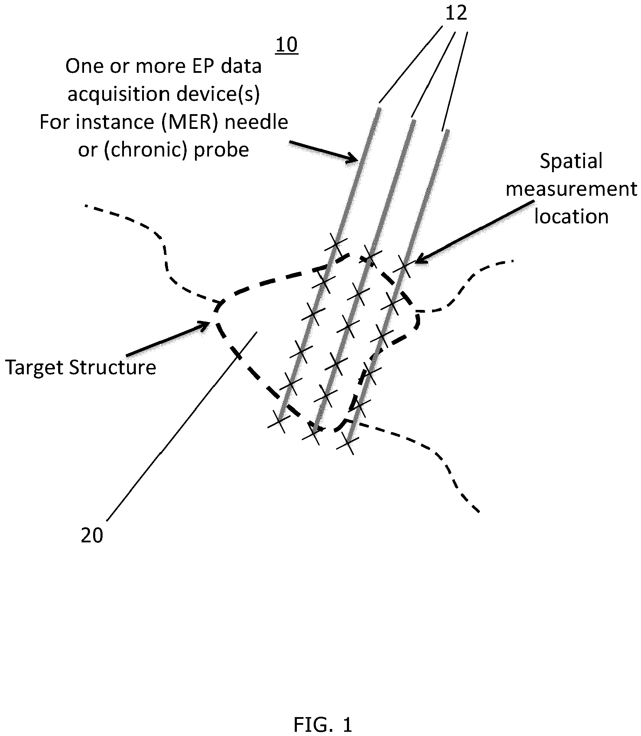

Figure 1 shows schematically that a number of measurement probes 12 are moved towards a target area 20 of a brain tissue and that different position measurements are taken.

The neurostimulation system 10 according to the present invention may consist of a computer program, an intra-operative electrophysiology (ioEP) system, and an implanted neurostimulation probe 100 connected to a unit capable of stimulation and signal-recording.

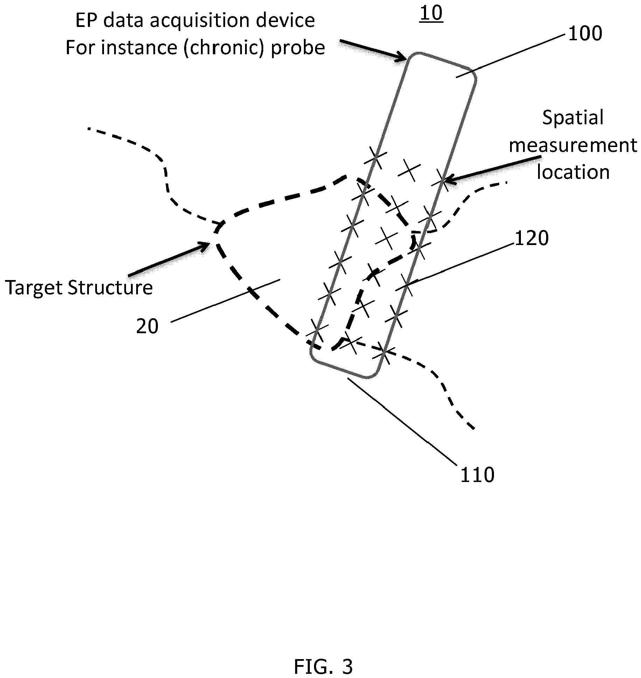

The measurement probes 12 are part of the ioEP system and are acutely implanted test-probes 12. The intra-operatively acquired data may be recorded either from acutely implanted test-probes 12, or from the chronic probe 100 (see e.g. figure 3) during implantation or both and may comprise recordings of electrical brain activity from the electrodes 120 of the probe 100 and/or stimulation-effects elicited by stimulation of particular electrode(s) or group(s) of electrodes.

The ioEP system is used during surgery to capture electrophysiological tissue characteristics at various positions in the brain. The computer program analyses these data and may construct as first data a set of intra-operatively acquired electrophysiological data, which is a 'map' of certain characteristic signal features.

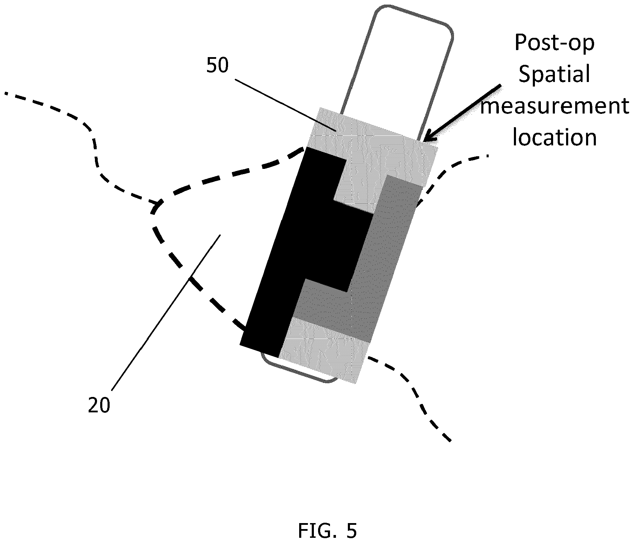

For example, the ioEP could have measured LFP signals and from this a 3D spatial map of LFP beta-activity (beta activity is a measure for oscillatory power in the 'beta-band', i.e. 15 - 30 Hz frequency band) is constructed. This map 40 allows to identify the boundaries of the functional target (target = high beta). In a later (post-op) phase, LFP may be recorded from the chronically implanted probe 100. This allows to construct a second spatial map 50 of LFP beta-activity (see figure 5).

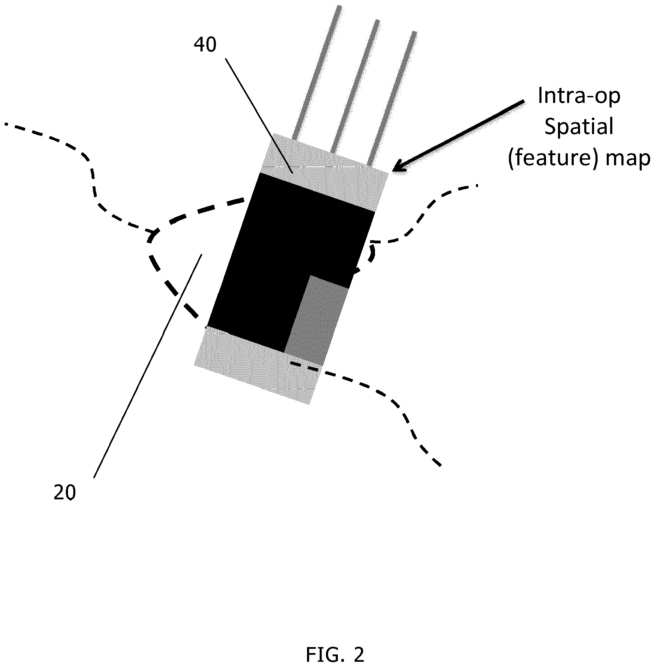

As shown in figure 2, from this intra-operatively acquired first set of data a spatial map 40 of certain signal characteristics related to the target area 20 is created and stored in storage means of the neurostimulation system 10.

Furthermore, as shown in figure 3, a chronic probe 100 of the neurostimulation system 10 is implanted at a certain position 110 in the target area 20. A plurality of electrodes 120 is arranged on a chronic probe 100, whereby the electrodes 120 are arranged in a predetermined geometrical manner forming an array on the probe 100.

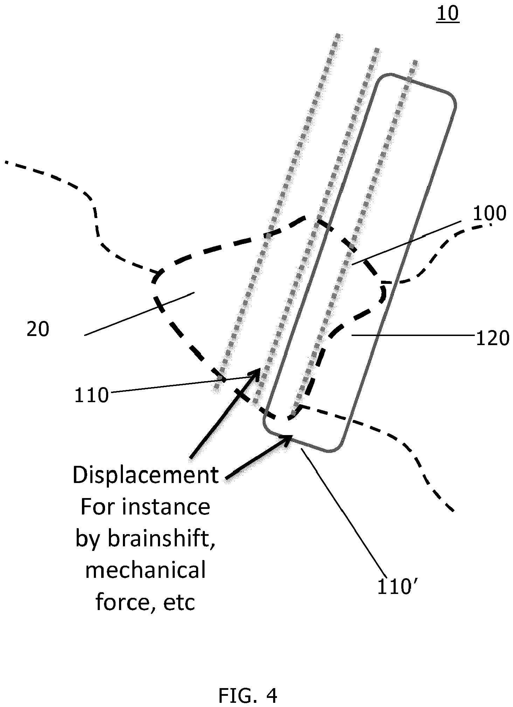

As shown in figure 4, over the time the probe 100 may get displaced and may have a different, unknown orientation with respect to the target area 20 and a new probe position 110' being different from the former position 110. It is always to consider that in the post-operative phase the relative position of the chronic probe 100 and its electrodes with respect to the target area 20 may change. This can have various causes, e.g. that immediately following to the probe implantation the surgical handling (e.g. fixation) causes some mechanical forces on the chronic probe 100 resulting in displacements or that in the post-surgery recovery period, the tissue will deform (recover from brain-shift), resulting in a shift of tissue with respect to the chronic probe 100 or that probe-displacements may occur over time, especially after strong forces applied on the lead (wire) or head.

As shown in figure 5, by performing a number of measurements, a new map 50 (also Post-op (feature) map 50) of signal characteristics can be created. The post-operatively acquired data from the chronic probe 100 may comprise recordings of electrical brain activity from the electrodes 120 of the probe 100 and/or stimulation-effects elicited by stimulation of particular electrode(s) or group(s) of electrodes.

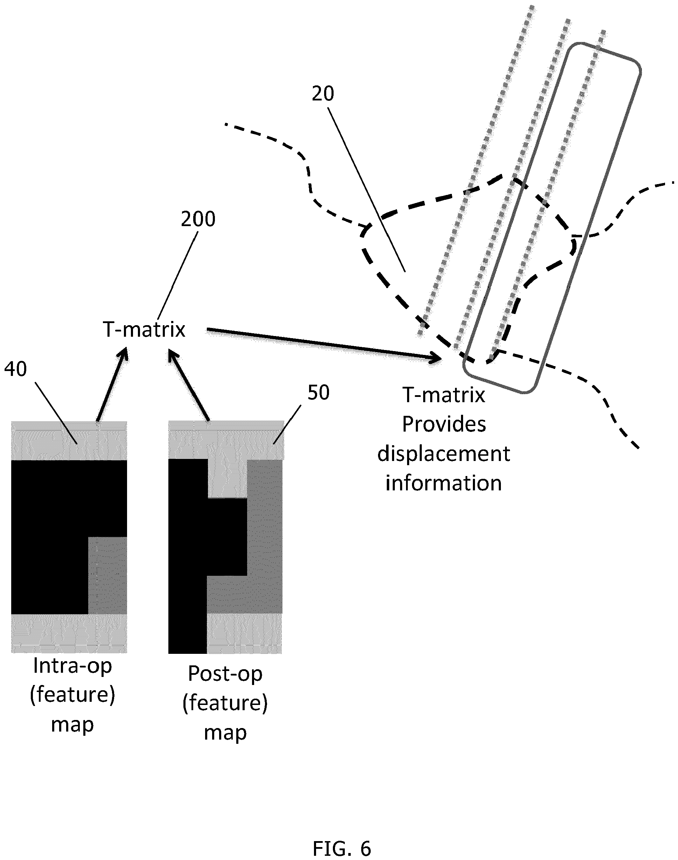

Furthermore, as shown in figure 6, a transformation matrix 200 is computed by comparing both maps 40, 50. This transformation matrix 200 may be used to transform other data so that the new probe position 110' (see figure 4) can be derived.

The two maps may be compared to each other, and by means of a spatial transformation (e.g. translation, rotation), the ioEP-map 40 (see figure 2; also so-called Intra-op (feature) map 40) can be fitted to the chronic-map 50 (see figure 5; also so-called Post-op (feature) map 50). Then, the functional boundaries determined during surgery are placed in the context of the chronic situation, and the target area 20 with respect to the chronic probe 100 is known.

It is possible that the same transformation matrix 200 can also be applied to other patient-information (e.g. an intra-operative image).

In an improved and/or alternative embodiment of the present invention, the intra-operative measurement and the post-operative measurement is made with the same (chronic) probe 100. In this case the chronic probe 100 is measured during surgical implantation with the goal to create an electrophysiological map (EP-map) of the target area 20. Then, the probe 100 is connected to an implantable pulse generator unit (IPG), the patient is closed and the surgery is finalized. In the post-operative phase, from the same probe 100 signals are recorded (using the IPG), and a post-op EP-map is constructed. By comparing the intra-op to the post-op EP-map, corrections can be made on target localization to the probe, e.g. to compensate a brain shift in the post-operative phase.

## Claims

1. A neurostimulation system (10) comprising at least one electrode (120) configured such that a tissue, advantageously a brain tissue, can be stimulated, storage means for storing at least a first and a second set of electrophysiological data, whereby the first set of data is a set of intra-operatively acquired electrophysiological data and whereby the second set of data is a set of a post-operatively acquired electrophysiological data, comparison means configured such that the first set of data and the second set of data can be compared to receive a set of comparison data, and analyzing means configured such that the set of comparison data can be at least partially analyzed to determine the position of the at least one electrode (120) or to determine data, from which the position of the at least one electrode (120) can be determined.

2. The neurostimulation system (10) according to claim 1,
**characterized in that**
the neurostimulation system (10) comprises at least one probe (100), whereby the probe (100) is equipped with the at least one electrode (120).

3. The neurostimulation system (10) according to claim 2,
**characterized in that**
the probe (100) is equipped with a plurality of electrodes (120), whereby the plurality of electrodes (120) is arranged in predetermined geometrical manner and whereby the electrodes (120) are configured such that the electrodes (120) are capable of providing electrical stimulation to the tissue and each of the electrodes (120) are capable of detecting electrical signals.

4. The neurostimulation system (10) according to claim 2 or 3,
**characterized in that**
the probe (100) is a chronic probe (100) configured such that the probe (100) is a long-term implantable probe (100).

5. The neurostimulation system (10) according to any of the claims 2 to 4,
**characterized in that**
the neurostimulation system (10) comprises test-probes (12), which are intra-operatively acutely implantable.

6. The neurostimulation system (10) according to any of the preceding claims,
**characterized in that**
the intra-operatively acquired data comprise recordings of electrical brain activity from the at least one electrode (120), preferably the electrode (120) of the probe (100), especially from a plurality of electrodes (120) and/or stimulation-effects elicited by stimulation of the at least one electrode (120), preferably from a plurality of electrodes (120), especially from a group of preselected electrodes (120).

7. The neurostimulation system (10) according to any of the preceding claims,
**characterized in that**
the post-operatively acquired data comprise recordings of electrical brain activity from the at least one electrode (120), preferably the electrode (120) of the probe (100), especially from a plurality of electrodes (120) and/or stimulation-effects elicited by stimulation of the at least one electrode (120), preferably from a plurality of electrodes (120), especially from a group of preselected electrodes (120).

8. The neurostimulation system (10) according to any of the preceding claims,
**characterized in that**
the neurostimulation system (10) comprises controlling means configured such that the neurostimulation system (10) can be controlled to acquire the at least first and second set of electrophysiological data.

9. The neurostimulation system (10) according to any of the preceding claims,
**characterized in that**
the neurostimulation system (10) comprises intra-operative electrophysiology system means configured such that intra-operatively electrophysiological tissue characteristics at various positions in the brain can be captured.

10. The neurostimulation system (10) according to claim 9,
**characterized in that**
the intra-operative electrophysiology system means comprise connection means configured such that intra-operative electrophysiology system means can be connected with the at least one electrode (120).

11. The neurostimulation system (10) according to claims 9 or 10,
**characterized in that**
the neurostimulation system (10) comprises data processing means configured such that data captured by intra-operative electrophysiology system means can be analyzed and that at least partially the first set of data can be provided by the data processing means.

12. The neurostimulation system (10) according to any of the preceding claims,
**characterized in that**
the first set of data being a set of intra-operatively acquired electrophysiological data is a first 3D spatial map of local-field potential beta-activity and that the second set of data being a set of a post-operatively acquired electrophysiological data is a second 3D spatial map of local-field potential beta-activity.

13. The neurostimulation system (10) according to claim 12,
**characterized in that**
the neurostimulation system (10) is configured such that the first 3D spatial map and the second 3D spatial map are compared by the comparison means and that the analyzing means are configured such that the analyzing means use a spatial translation to determine the position of the at least one electrode (120) or to determine data, from which the position of the at least one electrode (120) can be determined.

14. The neurostimulation system (10) according to claim 13,
**characterized in that**
the analyzing means are configured such that by means of the spatial translation functional boundaries determined intra-operatively are placed in the context of the chronic situation of the at least one electrode (120).

15. The neurostimulation system (10) according to claim 13 or 14,
**characterized in that**
the analyzing means are configured such that the analyzing means use a spatial translation to process additional patient information data, especially an intra-operative image, preferably an image acquired by magnetic resonance imaging.
